# EUROPEAN PATENT SPECIFICATION

(11) **EP 1 303 295 B1**
(45) Date of publication and mention of the grant of the patent: **13.09.2006**
(21) Application number: 01967094.2
(22) Date of filing: 28.06.2001
(51) Int. Cl.: A61K 38/17, A61P 17/00, A61P 31/04, A61P 31/10

(54) **CASEINOGLYCOMACROPEPTIDE FOR INHIBITING ADHESION OF THE PATHOGENIC FLORA OF THE SKIN**
CASEINOGLYCOMACROPEPTIDE ZUR INHIBIERUNG DER ADHÄSION VON PATHOGENER FLORA AUF DER HAUT
CASEINOGLYCOMACROPEPTIDE POUR INHIBER L'ADHESION DE LA FLORE PATHOGENE DE LA PEAU

(30) Priority: 14.07.2000 EP 00115274
(43) Date of publication of application: 23.04.2003
(73) Proprietor: SOCIETE DES PRODUITS NESTLE S.A., 1800 Vevey (CH)
(72) Inventor: NEESER, Jean-Richard, CH-1073 Savigny (CH); AUZANNEAU, Isabelle, F-06650 Opio (FR)
(74) Representative: Thomas, Alain
(86) International application number: PCT/EP2001/007293
(87) International publication number: WO 2002/005839

(56) References cited:
- WO-A-95/32728
- WO-A-98/46240
- FR-A- 2 496 465
- FR-A- 2 657 525
- FR-M- 8 087
- US-A- 3 764 670
- US-A- 4 007 264
- US-A- 4 525 351
- US-A- 4 952 560
- US-A- 5 166 132
- US-A- 5 681 586
- CHERKAOUI S ET AL: "DETERMINATION OF THE STABILITY OF CASEINOGLYCOMACROPEPTIDE IN A COSMETIC LOTION BY USE OF CAPILLARY ZONE ELECTROPHORESIS WITH A COATED CAPILLARY" CHROMATOGRAPHIA, vol. 50, no. 5/6, September 1999 (1999-09), pages 311-316,

## Description

The subject of the present invention is the use of caseinoglycomacropeptide (CGMP), aglycosylated derivative of CGMP or asialo CGMP for the preparation of a composition for preventing or treating disorders induced by the pathogens of the cutaneous system.

### State of the art

The proliferation of pathogens such as *taphylococcus aureus, Streptococcus pyogenes* or *Propionibacterium acnes,* or of some yeasts such as *Candida albicans,* can cause dysregulation of the cutaneous system or even more serious disorders in the skin or the mucous membranes such as eczema, candidiasis, dermatitis, and the like.

Numerous means of treatment against these pathogenic agents are known. Antibiotics or chemical antibacterial agents and antifungals are the most conventionally used. Alternatively, topical disinfectants of the aldehyde type are chosen.

Thus, published patent application FR 2740039 describes the use of a substance chosen from aldehydes and bifunctional compounds, preferably glutaraldehyde, for inhibiting the attachment of pathogenic strains such as *Staphylococcus aureus* to the keratinocytes and corneocytes.

Also, hexachlorophene and its derivatives are known as antibacterial substances and are more particularly used against *Propionibacterium acnes.* These treatments are in general expensive and harmful both to health and to the environment.

Alternative, nontoxic treatments are also known which consist in using the antifungal, bactericidal or bacteriostatic properties of certain strains of microorganisms.

Thus, application EP 99204489 describes the use of strains of *Lactobacillus, Micrococcus* or *Bifidobacterium* selected for their adhesion capacity with respect to skin cells and their competitive properties with respect to the adhesion of the pathogens of the cutaneous system, to prepare cosmetic compositions capable of stabilizing and regulating the pathogenic flora of the skin.

Also, application PCT/EP96/00441 describes sugars and their derivatives as antiadhesion active agents for the treatment of yeast and dermatophyte mycoses.

The invention is intended to find a novel natural nonbacterial agent capable of controlling and regulating the cutaneous ecosystem.

### Summary of the invention

Thus, the present invention relates to the use of a casein derivative for the preparation of a composition for preventing or treating disorders induced by the pathogens of the cutaneous system, said casein derivative being chosen from the group comprising caseinoglycomacropeptide (CGMP), the glycosylated derivatives of caseinoglycomacropeptides and Asialo-CGMP.

Indeed, it has been observed, surprisingly, that casein derivatives were capable of stabilizing and/or regulating the pathogenic flora of the cutaneous system by inhibiting the adhesion of pathogens such as *Streptococcus pyogenes, Trichophyton rubrum, Pityrosporum ovale, M furfur* or *Candida albicans*, for example.

Caseinoglycomacropeptides (CGMPs) may be used in any form, and in particular in the form of calcium or sodium salts, for example.

A composition comprising CGMPs may be in particular in the form of a cream, lotion, dermatological cake, shampoo or powder, for example.

The quantity of CGMPs contained in the composition may be from 0.01% to 10% by weight, and preferably from 0.5% to 5% by weight of the dry matter content.

### Detailed description of the invention

The name "cutaneous system" covers all the cells of the skin (i.e. keratinocytes) but also the comeocytes.

According to a first object, the present invention proposes the use of at least one casein derivative selected from the group comprising caseinoglycomacropeptide (CGMP), a glycosylated derivative of caseinoglycomacropeptide or , for the preparation Asialo-CGMP of a composition for the purpose of preventing or treating disorders induced by pathogens of the cutaneous system.

Indeed, it has been observed, surprisingly, that caseinoglycomacropeptides (CGMPs) and their derivatives had the capacity to adhere to cutaneous cells, to stabilize and to regulate the pathogenic bacterial flora of the cutaneous system, in particular by inhibiting the adhesion of pathogens such as *Streptococcus pyogenes, Trichophyton rubrum, Pityrosporum ovale, Candida albicans, M furfur,* for example.

The cutaneous disorders induced by these pathogens may be in particular atopic dermatitis (in the remission phases as maintenance treatment), acne, candidiasis, seborrhoeic dermatitis, Pityriasis versicolor, impetigo or eczematous superinfections (Table 1).

**Table 1: Dermatological disorders caused by pathogens of the cutaneous system**

| **Pathogens** | Dermatological information |
|---|---|
| ***Streptococcus pyogenes*** | Impetigo, eczema and herpes superinfected |
| ***Candida albicans*** | Candidiasis |
| ***Pityrosporum ovale*** | Seborrhoeic dermatitis, Pityriasis versicolor |
| ***Trichophyton rubrum*** | Onychomycosis, dermatophytosis |
| ***M. furfur*** | Varied |

Disorders of the cutaneous system may also be linked to therapeutic treatments with antibiotics, with antimycotics, to diabetes (candidiasis), to a pathological condition of the mucous membranes (vaginal candidiasis), to chronic eczema (homeostasis disequilibrium), to sensitive skins (premature babies, children), greasy skins (linked to hormonal dysregulations which may promote the establishment of bacteria) or to dandruff states.

According to a preferred embodiment of the invention, the CGMPs may be used in the form of sodium or calcium salts, for example. It is also possible to use their derivatives, in particular their glycosylated (e.g. sialylated) derivatives or the active products of their hydrolysis.

The CGMPs may be used at concentrations ranging from 0.01 to 10%, and preferably from 0.5 to 5%.

CGMP, which is a sialylated macropeptide formed by the action of rennet or of chymosin on milk kappa-caseins, is preferably used. To prepare CGMP, it is possible to use an ion-exchange method of treating a whey raw material, for example as described in PCT 98/53702 which comprises the following steps: where appropriate, adjusting the pH of the liquid whey raw material to a value of 1 to 4.3, bringing the said liquid into contact with a predominantly weak anionic resin in alkaline form until a stabilized pH of 4.5-5.5 is obtained, and then separating the resin and the liquid whey product which is recovered, and desorbing the CGMP from the resin.

As whey raw material, there may be used any product or by-product containing CGMP, and for example:
- the sweet whey obtained after separation of casein coagulated with rennet,
- a sweet whey or such a whey which has demineralized to a greater or lesser degree for example by electrodialysis, ion-exchange, reverse osmosis, electrodeionization or a combination of these methods,
- a concentrate of sweet whey,
- a concentrate of sweet whey which has been demineralized to a greater or lesser degree for example by electrodialysis, ion-exchange, reverse osmosis, electrodeionization or a combination of these methods,
- a concentrate of substantially lactose-free sweet whey proteins obtained for example by ultrafiltration followed by diafiltration,
- lactose crystallization mother liquors from a sweet whey,
- a sweet whey ultrafiltration permeate,
- the product of hydrolysis, by a protease, of a native casein obtained by acid precipitation of skimmed milk by an inorganic acid or by biological acidification, where appropriate with addition of calcium ions,
- the product of hydrolysis of a caseinate by a protease.

To prepare such a composition, the CGMP is incorporated into a pharmaceutically or cosmetically acceptable carrier, in a quantity varying according to the desired application. It may be present at from 0.01 to 10% relative to the total weight of the composition, preferably from 0.5 to 5%.

The compositions may be administered by the topical or ocular route.

By the topical route, the pharmaceutical compositions based on compounds according to the invention are preferably intended for the treatment of the skin and of the mucous membranes and may be provided in the form of ointments, creams, milks, pomades, powders, impregnated pads, solutions, gels, sprays, lotions or suspensions. They may also be provided in the form of microspheres or nanospheres or lipid or polymeric vesicles or polymeric patches or hydrogels allowing controlled release. These compositions for topical administration may be provided either in anhydrous form or in aqueous form depending on the clinical indication.

By the ocular route, they are mainly collyria.

In the compositions, the CGMPs may be combined with retinoids or corticosteroids, or combined with anti-free radical agents, with α-hydroxy or α-keto acids or their derivatives, or with ion-channel blockers.

The pharmaceutical compositions may, in addition, contain inert or even pharmacodynamically or cosmetically active additives or combinations of these additives and in particular: wetting agents; depigmenting agents such as hydroquinone, azelaic acid, caffeic acid or kojic acid; emollients; moisturizing agents such as glycerol, PEG-400, thiamorpholinone and its derivatives or urea; antiseborrhoeic or anti-acne agents, such as S-carboxymethylcysteine, S-benzylcysteamine, their salts and their derivatives, or benzoyl peroxide; antibiotics such as erythromycin and its esters, neomycin, clindamycin and its esters, tetracyclines; antifungal agents such as ketoconazole or 4,5-polymethylene-3-isothiazolinones; agents promoting hair regrowth, such as Minoxidil (2,4-diamino-6-piperidinopyrimidine-3-oxide) and its derivatives, Diazoxide (7-chloro-3-methyl-1,2,4-benzothiadiazine 1,1-dioxide) and Phenytoïn (5,4-diphenyl-2,4-imidazolidinedione); nonsteroidal anti-inflammatory agents; carotenoids and, in particular, β-carotene; antipsoriatic agents such as anthralin and its derivatives and finally 5,8,11,14-eicosatetraynoic and 5,8,11-eicosatrynoic acids, their esters and amides.

The composition may also contain preservatives such as esters of para-hydroxybenzoic acid, stabilizing agents, moisture-regulating agents, pH-regulating agents, osmotic pressure-modifying agents, emulsifying agents, UV-A and UV-B screening agents, antioxidants such as α-tocopherol, butylated hydroxyanisole or butylated hydroxytoluene.

The composition is intended in particular for therapeutic or prophylactic treatment of diseased skins and/or mucous membranes which may exhibit disorders of the cutaneous system such as in particular:
- infectious complications such as superinfected atopic dermatitis, impetiginized eczema, ulcer, wounds, bums, superinfected inflammatory acne,
- polydermatites such as impetigo, superficial folliculites,
- seborrhoeic dermatites, Pityriasis versicolor
- dermatophytoses (Tinea capitis, Tinea corporis, athlete's foot, Hebra's eczema marginatum, Tinea circinata)
- candidiases (vaginal, interdigital, linked to risky professions or to diabetes)
- disorders linked to therapeutic treatments with antibiotics or with antimycotics,
- disorders caused by hormonal dysregulations (greasy skins) or dandruff states
- sensitive skins (premature babies, children).

A composition for veterinary use for animals, containing at least one CGMP derivative having the properties described above may be provided in the form of dry or liquid shampoos, powders, mousses or lotions for example. It may contain up to 10% CGMPs, for example.

The composition for veterinary use is intended for the treatment or prevention of infections caused by ectoparasites, dysfunctions due to streptococcal infections (due to *S. pyogenes*) and mycotic infections (candidiases due to *C. albicans* and pityrosporoses due to *P. canis*).

The veterinary composition may have anti-inflammatory, antiitching or antidandruff properties.

The present invention is described in greater detail below with the aid of the examples which are given by way of illustration of the subject of the invention and which do not constitute in any manner a limitation thereto. The percentages are given by weight unless otherwise stated.

### Examples

### Example 1: Preparation of CGMP and of As-CGMP

A sweet bovine whey is used which is concentrated beforehand to 17% dry matter content, and then demineralized by electrodialysis, decationized on a column of strong cationic resin, deanionized on a column of weak anionic resin and spray-dried in a drying tower, having the composition indicated below:

| | |
|---|---|
| Proteins (CGMP included) | 11.7% |
| Lactose | 81.7% |
| Ash | 1% |
| Lipids | 1% |
| Water | balance for 100 |

This demineralized whey powder is solubilized in deionized water and the solution has an initial pH of 3.8. In the above installation, 392 kg of this solution are treated at the temperature of 8°C by stirring it in the reactor in the presence of 23 kg of weak anionic resin (IMAC HP 661®, Rohm & Haas regenerated in OH⁻ form) for 4 h.

The stabilization of the pH at 4.89 indicates the end of the reaction. The liquid is then drawn off and the resin is recovered as above. After concentrating the liquid to 28% dry matter content by evaporation, the concentrate is spray-dried in a drying tower.

An analysis of the concentrate by HPLC shows that the reaction removed 89% of the initial CGMP. Moreover, the powder contains 9.1% of whey proteins, which corresponds to a yield of 90% of whey proteins.

To recover the CGMP, the resin is successively washed with deionized water, with 301 of a 0.5% aqueous HCl solution and with 301 of deionized water, and then the CGMP is eluted with twice 401 of a 2% aqueous NaOH solution and rinsed with 301 of deionized water. After having combined the eluate and rinsing volumes, the whole is concentrated to a volume of 251 1 by ultrafiltration with a membrane having a nominal cutoff of 3000 daltons, and then the retentate is freeze-dried and 900 g of CGMP are obtained, which corresponds to a yield of 80% relative to the initial CGMP.

The Asialo-CGMP (As-CGMP) is obtained by enzymatic or chemical desialylation, as described by Neeser J.R. et al., 1988, *Infect. Immun*., **56**, 3201-3208.

### Example 2: Tests in vitro for inhibiting the adhesion of Streptococcus pyogenes, Trichophyton rubrum and Candida albicans with the casein derivatives CGMP and As-CGMP.

The *in vitro* adhesion model is based on the incubation of a radiolabelled and standardized suspension of a pathogenic cutaneous microorganism with a monolayer of immortalized human keratinocytes (HaCaT line, Boukamp P. *et al*., *J. Cell Biol*., **106**, 761-771, 1988).

The inhibitory activity of CGMP in relation to this adhesion is evaluated in the context of a coincubation with the monolayer of the pathogen and of the compound to be tested by assaying the radioactivity retained on the monolayer.

### Keratinocytes

The HaCaT cells are cultured in DMEM medium supplemented with 10% foetal calf serum, at 37°C under 5% CO₂. They are inoculated in a 6-well cluster at the rate of 1.0E+04 cells/cm². The adhesion trial is carried out 5 days after confluence. The monolayers are washed 3 times with PBS before incubation with the microorganisms.

### Microorganisms

The pathogens *Streptococcus pyogenes, Trichophyton rubrum* and *Candida albicans* are cultured in broth by subculturing from a slope culture according to standard procedures which are specific to them. An OD/bacterial density relationship was established for each of the microorganisms tested, on the basis of the serial dilutions and the counts on agar medium.

### Radiolabelling

The radiolabelling of the microbial strains is obtained by incorporating 100 µCi/10 ml of ³H-adenine for 24 h of culture into TCS broth. The suspension is then centrifuged for 10 minutes at 3000 rpm and washed 3 times in PBS. The cell density is adjusted with PBS buffer to about 2.0E+08 cfu/ml (OD at 525 nm = 0.5). The specific radioactivity is determined by scintillation counting on 100 µl of the suspension.

### Adhesion assay:

1 ml of the radiolabelled microbial suspension is incubated for 1 h at 35°C. The monolayer is washed 3 times with PBS buffer and lysed by addition of 1 N NaOH for 30 minutes at room temperature. The lysate is transferred to a scintillation flask and incubated for 1 h at 60°C with 1 ml of hyamine hydroxide (Carlo Erba, ref. 464951). The ³H activity is counted in a liquid scintillation counter. Each assay is repeated in triplicate. A control for adhesion to the plastic support is also carried out.

Adhesion is defined as the ratio of the radioactivity adhered to the radioactivity introduced, multiplied by 100.

### Adhesion inhibition assay

The solutions of casein derivatives are prepared in PBS. The concentrations tested cover the range 0-10 mg/ml. The configuration tested consists in simultaneously incubating the radiolabelled pathogen and the derivative on the monolayer.

The results are given in Tables 2, 3 and 4.

**Table 2: Inhibition of the adhesion of S. pyogenes to HaCaT cells by various concentrations of CGMP and As-CGMP.**

| Concentrations | Inhibition of *S. pyogenes* adhesion (%) | | | | | |
|---|---|---|---|---|---|---|
| | 1 mg/ml | 2 mg/ml | 3 mg/ml | 5 mg/ml | 8 mg/ml | 10 mg/ml |
| CGMP | 34 | 45 | 52 | 55 | 58 | 60 |
| As-CGMP | 15 | 27 | 35 | 52 | 60 | 65 |

**Table 3: Dose/effect of CGMP on the adhesion of S. pyogenes, C. albicans to the keratinocytes HaCaT in culture.**

| CGMP concentration | Adhesion (%) | | | | | |
|---|---|---|---|---|---|---|
| | 0 mg/ml | 1 mg/ml | 2 mg/ml | 5 mg/ml | 7 mg/ml | 10 mg/ml |
| ***S. pyogenes*** | 100 | 77 | 43 | 37 | 30 | 23 |
| ***C. albicans*** | 100 | 68 | 61.5 | 61.5 | 58 | 52 |

**Table 4: Dose/effect of CGMP on the adhesion of T. rubrum to the keratinocytes HaCaT in culture.**

| CGMP concentration | Spores/100 keratinocytes | | | | | |
|---|---|---|---|---|---|---|
| | 0 mg/ml | 1 mg/ml | 2 mg/ml | 5 mg/ml | 7 mg/ml | 10 mg/ml |
| ***T. rubrum*** | 88 | 87 | 86 | 68 | 40 | 1 |

CGMP and As-CGMP have the capacity to inhibit the adhesion of pathogens such as *Streptococcus pyogenes, Trichophyton rubrum, Candida albicans,* for example. They can thus stabilize or regulate the pathogenic bacterial flora of the cutaneous system.

### Example 3: Tests ex vivo for inhibition of the adhesion of M. furfur by CGMP.

### Materials and methods

**Animals:** 15 SKH female mice, 7 to 8 weeks old and weighing about 30 g, were provided by C. River. 5 mice were used for each group testing a different topical application.

### Preparation of the inoculum

A suspension of *M. furfur* is prepared for its inoculation in mice. For that, a slope preculture of strain 1 is carried out on a solid medium (AES, AEB 122 859) at 35°C for 18 to 24 h. After incubation, the bacterium is resuspended in 10 ml of sterile saline solution and then recovered after centrifugation at 3000 rpm for 10 min. The supernatant is then removed and the pellet is taken up in 10 ml of saline solution. This procedure is repeated twice. An inoculum suspension is prepared by resuspending the bacteria washed in 4 ml of sterile saline solution. The OD at 525 nm is adjusted to about 0.14. It contains about 10⁸ cfu/ml.

### Inoculation of the mice

The skin of the mice is delipidized on the flanks with 95% ethanol (Merck). 50 µl of a suspension containing a 50/50 mixture of the inoculum 1.0E+07 cfu/ml and of the product to be tested (CGMP in solution at 1%, 2%, 3% and 5%) were slowly applied with the aid of a micropipette over the delipidized area (6.25 cm²). The inoculated sites are protected by occlusion for 1 h under a sterile plastic dressing (Dermafilm 33 x 15, ref. 38.3015, Vygon laboratory).

### Counting of the viable bacteria in the lesions

4 hours after the application of the suspension, the mice are killed under anaesthesia with forene (Abbott France). The inoculated sites are excised as a block (diameter 12 mm). The skin biopsies removed are ground and homogenized in 2 ml of sterile saline solution with a Polytron (PT 2100, Bioblock Scientific) (5 rpm, 5 min).

A 1 ml sample of homogenized tissue is added to 9 ml of a sterile saline solution and 0.1 ml of this mixture is cultured on medium No. 110 using the 10-fold dilution method. After incubating for 48 hours at 35°C, the colonies developed are counted and the CFU (colony forming units) are determined.

### Results

The results are presented in Table 5.

**Table 5: Adhesion of M. furfur in the presence of various concentrations of CGMP.**

| CGMP concentration in solution | Adhesion (% of the control) | | | | |
|---|---|---|---|---|---|
| | Control | 1% | 2% | 3% | 5% |
| ***M. furfur*** | 100 | 32 | 22 | 16 | 15 |

### Example 4: Body lotion

A body lotion is prepared which has the following composition: 8.0% mineral oil, 5.0% isopropyl palmitate, 2.0% polyglyceryl-3 diisostearate, 4.0% octyldodecanol, 0.3% carbomer, 0.2% sodium cocoylglutamate, 1.2% sodium hydroxide at 10%, a preservative, perfume, 0.5 to 5% CGMP as prepared in Example 1. The mixture is adjusted to 100% with water.

The body lotion thus obtained, by virtue of its antiadhesion properties against pathogens, is intended to stabilize and/or to regulate the pathogenic skin flora.

### Example 5: Shampoo

A shampoo is prepared which has the following composition: 7% sodium lauryl sulphate, 2% cocamidopropylbetain, 2% sodium lauryl sulphonosuccinate, sodium chloride, preservative, perfume and 3% CGMP as prepared in Example 1. Their mixture is adjusted to 100% with water.

The shampoo thus prepared has properties which regulate the pathogenic scalp flora. It is in particular indicated in the treatment of dandruff states.

### Example 6: Pharmaceutical composition

To obtain a pharmaceutical composition having properties which regulate the pathogenic skin flora, the fatty and aqueous phases having the following composition are prepared:

| | | |
|---|---|---|
| | CGMP as prepared in Example 1 | 1 % |
| Fatty phase: | Arachidyl behenyl | |
| | alcohol/arachidylglucoside | 3 % |
| | Isohexadecane | 7% |
| | Sweet almond oil | 3% |
| | Shea butter | 2% |
| | B.H.T. | 0.05% |
| | Propyl POB | 0.05% |
| Aqueous phase: | Water | qs 100% |
| | Glycerin | 5% |
| | Methyl POB | 0.1% |

The fatty and aqueous phases are heated to 75°C. Emulsification is then carried out by adding the aqueous phase to the fatty phase with stirring using a Rayneri device at 1000 rpm. 30 minutes after the emulsification, the mixture is homogenized for 1 minute using a Polytron (speed 4-5).

### Example 7: Pharmaceutical composition

A composition is prepared in the same manner as in Example 6, but having the following composition:

| | | |
|---|---|---|
| | CGMP as prepared in Example 1 | 1% |
| Fatty phase: | glyceryl stearate and PEG 100 stearate | 5% |
| | Isohexadecane | 8% |
| | Shea butter | 5% |
| | B.H.T. | 0.05% |
| | DC 1503 | 1% |
| Aqueous phase: | Water | qs 100% |
| | Glycerin | 3% |
| | Carbopol 981 | 0.2% |
| | Lubrajel | 5% |
| | Phenoxyethanol | 1% |
| | Sodium hydroxide | qs pH 6 |

### Example 8: Shampoo for animals

A shampoo for animals is prepared which has the following composition: 5% sodium lauryl sulphate, 2% cocamidopropylbetain, 2% sodium lauryl sulphonosuccinate, 2% sodium chloride, 1.5% PEG-7 Glyceryl Cocoate, 0.75% propylene glycol, panthenol, glycerin, disodium phosphate, preservative, perfume and 2% CGMP as prepared in Example 1. The mixture is adjusted to 100% with water.

The shampoo thus prepared has properties which regulate the pathogenic flora of the cutaneous system of animals.

## Claims

1. Use of a casein derivative for the preparation of a composition for preventing or treating disorders induced by the pathogens of the cutaneous system, in which the casein derivative is caseinoglycomacropeptide (CGMP), a glycosylated derivative of caseinoglycomacropeptide or Asialo CGMP.

2. Use according to Claim 1, in which the casein derivative is capable of stabilizing and/or regulating the pathogenic flora of the cutaneous system by inhibiting the adhesion of pathogens such as *Streptococcus pyogener, Trichophyton rubrum, Pityrosporum ovale, Candida albicans* or *M. furfur*.

3. Use according to Claim 1 or 2, in which the casein derivative is used in an amount of 0.01 to 10% by weight.

4. Use according to one of Claims 1 to 3, in which the disorders of the cutaneous system are:
- infectious complications such as superinfected atopic dermatitis, impetiginized eczema, ulcer, wounds, bums, superinfected inflammatory acne,
- polydermatites such as impetigo, superficial folliculites,
- seborrhoeic dermatites, Pityriasis versicolor
- dermatophytoses
- candidiases
- those linked to therapeutic treatments with antibiotics or with antimycotics,
- those caused by hormonal dysregulations or dandruff states
- dysfunctions linked to streptococcal and mycotic infections, ectoparasites, inflammation, itching or dandruff states in animals.

## Patentansprüche

1. Verwendung eines Caseinderivats zur Herstellung einer Zusammensetzung zur Prävention oder Behandlung von Erkrankungen, die durch Pathogene des Hautsystems ausgelöst werden, wobei das Caseinderviat Caseinoglykomacropeptid (CGMP), ein glykolysiertes Derivat von Caseinoglykomakropeptid oder Asialo-CGMP ist.

2. Verwendung nach Anspruch 1, wobei das Caseinderivat in der Lage ist, die pathogene Flora des Hautsystems **dadurch** zu stabilisieren und/oder zu regulieren, dass die Adhäsion von Pathogenen wie *Streptococcus pyogenes, Trichophyton rubrum, Pityrosporum ovale, Candida albicans* oder *M. furfur* inhibiert wird.

3. Verwendung nach Anspruch 1 oder 2, wobei das Caseinderivat in einer Menge von 0,01 bis 10 Gew.-% verwendet wird.

4. Verwendung nach einem der Ansprüche 1 bis 3, wobei die Erkrankungen des Hautsystems sind:
- infektiöse Komplikationen wie eine superinfizierte atopische Dermatitis, impetiginisierte Ekzeme, Wunden, Verbrennungen, superinfizierte inflammatorische Akne,
- Polydermatitis wie Impetigo, oberflächliche Folliculitis,
- seborrhoische Dermatitis, Pityriasis versicolor,
- Dermatophytosen,
- Candidiasis,
- solche, die mit therapeutischen Behandlungen mit Antibiotika oder mit Antimykotika verknüpft sind,
- solche, die durch hormonale Fehlsteuerungen oder Schuppenzustände bewirkt werden,
- Funktionsstörungen, die mit Streptokokken- und Pilzinfektionen, Ectoparasiten, Entzündung, Jucken oder Schuppenzuständen bei Tieren verbunden sind.

## Revendications

1. Utilisation d'un dérivé de caséine pour la préparation d'une composition destinée à empêcher ou à traiter des troubles induits par les agents pathogènes du système cutané, dans laquelle le dérivé de caséine est un caséinoglycomacropeptide (CGMP), un dérivé glycosylé d'un caséinoglycomacropeptide ou un Asialo-CGMP.

2. Utilisation selon la revendication 1, dans laquelle le dérivé de caséine est capable de stabiliser et/ou de réguler la flore pathogène du système cutané en inhibant l'adhésion d'agents pathogènes tels que *Streptococcus pyogenes, Trichophyton rubrum, Pityrosporum ovale, Candida albicans* ou *M. furfur.*

3. Utilisation selon la revendication 1 ou 2, dans laquelle le dérivé de caséine est utilisé en une quantité allant de 0,01 à 10% en poids.

4. Utilisation selon l'une des revendications 1 à 3, dans laquelle les troubles du système cutané sont :
- des complications infectieuses telles que la dermite atopique surinfectée, l'eczéma impétigineux, les ulcères, les plaies, les brûlures, l'acné inflammatoire surinfectée,
- des polydermites telles que l'impétigo, les folliculites superficielles,
- la dermite séborrhéique, le pityriasis versicolor,
- les dermatophytoses,
- les candidoses,
- ceux liés aux traitements thérapeutiques par des antibiotiques ou par des antifongiques,
- ceux provoqués par des dérèglements hormonaux ou des états pelliculaires,
- les dysfonctionnements liés aux infections streptococciques et fongiques, aux ectoparasites, aux inflammations, aux prurits ou aux états pelliculaires chez l'animal.
